# FASCICULE DE BREVET EUROPEEN

(11) **EP 4 681 130 B1**
(45) Date de publication et mention de la délivrance du brevet: **21.10.2026**
(21) Numéro de dépôt: 24712030.6
(22) Date de dépôt: 14.03.2024
(51) Int. Cl.: G06Q 10/047, G06Q 10/0631, G06Q 50/40, G16H 40/00, G16H 40/20

(54) **GESTION OPTIMISÉE DES INTERVENTIONS ENTRE AU MOINS DEUX INDIVIDUS SUR UN MÊME LIEU D'INTÉRÊT**
OPTIMIERTE VERWALTUNG VON EINGRIFFEN ZWISCHEN MINDESTENS ZWEI PERSONEN AN DERSELBEN STELLE VON INTERESSE
OPTIMISED MANAGEMENT OF INTERVENTIONS BETWEEN AT LEAST TWO INDIVIDUALS AT THE SAME PLACE OF INTEREST

(30) Priorité: 17.03.2023 FR 2302503
(43) Date de publication de la demande: 21.01.2026
(73) Titulaire: IAVC, 59120 Loos (FR)
(72) Inventeur: ROUSSEAUX, Adrien, 59000 Lille (FR); BUSSIÈRE, Pierre-Olivier, 59910 Bondues (FR)
(74) Mandataire: RVDB
(86) Numéro de dépôt international: PCT/EP2024/056802
(87) Numéro de publication internationale: WO 2024/194138

(56) Documents cités:
- JP-A- 2021 026 649
- US-A1- 2019 120 643
- AFIFI SOHAIB ET AL: "Heuristic solutions for the vehicle routing problem with time windows and synchronized visits", OPTIMIZATION LETTERS, SPRINGER BERLIN HEIDELBERG, BERLIN/HEIDELBERG, vol. 10, no. 3, 26 March 2015 (2015-03-26), pages 511 - 525, XP035715362, ISSN: 1862-4472, [retrieved on 20150326], DOI: 10.1007/S11590-015-0878-3

## Description

### Domaine technique

L'objet de la présente invention concerne le domaine de la gestion des interventions (planifiées ou non-planifiées) entre au moins deux individus.

Un des objectifs de la présente invention est de fournir une solution pour synchroniser les arrivées respectives d'au moins deux individus sur un même lieu d'intérêt, encore appelé lieu d'intervention.

Un des objectifs de la présente invention porte plus particulièrement sur la synchronisation des arrivées respectives de plusieurs personnes ayant besoin d'arriver en un même point géographique au même instant ou à des instants décalés.

L'objet de la présente invention trouvera des applications avantageuses dans de nombreux domaines dont notamment la santé en permettant de gérer les interventions, et ce qu'il s'agisse d'interventions planifiées ou non-planifiées.

La présente invention trouvera par exemple une application particulièrement avantageuse dans l'organisation des professionnels de santé pour la prise en charge d'un patient dans une situation d'urgence comme par exemple la prise en charge d'un patient victime d'un AVC (pour Accident Vasculaire Cérébral).

On comprendra que la présente invention trouvera des applications pour la prise en charge d'autre type de patients et s'appliquera de façon générale à la gestion optimisée de toutes les interventions nécessitant à la fois l'acheminement d'un patient vers un établissement de santé et l'arrivée synchronisée d'au moins un professionnel de santé sur le lieu d'intervention. Bien évidemment, la présente invention ne s'appliquera pas exclusivement au domaine de la santé, mais trouvera également des applications avantageuses dans d'autres domaines comme par exemple le covoiturage ou encore la logistique de colis.

### Art antérieur

Bien qu'il s'agisse d'un sujet de santé publique majeur, le Demandeur observe que la prise en charge d'un patient dans une situation d'urgence n'est aujourd'hui pas optimisée.

Les solutions mises en œuvre aujourd'hui restent en effet rudimentaires.

Traditionnellement, il existe pour les situations d'urgence :
- des systèmes permettant de géolocaliser les individus ;
- des systèmes permettant de partager des données de géolocalisation ;
- des systèmes permettant de calculer les temps de trajet d'un premier point vers un deuxième point.

En connaissant l'heure et le point de départ d'un patient, les professionnels de santé peuvent donc calculer à un instant déterminé le temps de trajet du patient et son propre temps de trajet. Pour obtenir ces informations (pour autant qu'ils arrivent à les obtenir), les professionnels de santé échangent :
- par téléphone avec le Service d'Aide Médicale Urgente (SAMU),
- entre professionnels de santé par téléphone ou des outils de communication numérique qui ne sont pas toujours sécurisés (généralement *WhatsApp*^{®}*).*

Les informations recueillies (lorsqu'elles sont recueillies et correctes) permettent de calculer manuellement l'heure à laquelle le ou les praticiens doivent quitter leur lieu de situation (domicile ou autre) pour arriver sur le lieu d'intervention.

En revanche, on sait pertinemment qu'un transport d'urgence du type SMUR (Service Mobile d'Urgence), ASSU (Ambulance de secours et de soins d'urgence) ou véhicule de pompier ne respecte pas le code de la route. Pour être suffisamment juste, ce calcul doit être mis à jour régulièrement en fonction du lieu où se trouve ce transport, et donc le patient. Ceci n'est actuellement pas possible.

Par ailleurs, on constate que la communication entre les intervenants dans une situation d'urgence est bien souvent omise pour des raisons humaines et/ou organisationnelles. Prenons l'exemple d'un patient victime d'un AVC.

On sait qu'un AVC est une maladie grave entraînant un déficit neurologique lié à des lésions cérébrales d'origine vasculaire.

Les premières heures de prise en charge sont décisives pour le patient.

*« Time is brain »* : en cas de survenance d'un AVC ischémique, à chaque minute, 2 millions de neurones disparaissent, et toutes les 30 minutes qui passent représentent 15% de handicaps supplémentaires non récupérables.

Dans certains cas, le patient doit recevoir une thrombectomie mécanique dans un centre de recours spécialisé, laquelle consiste à déboucher l'artère cérébrale à l'origine de l'AVC afin de rétablir la circulation sanguine et ainsi limiter les lésions.

Dans ce cas, le patient qui est pris en charge par les services d'urgence doit être transféré dans les meilleurs délais depuis le premier établissement de santé vers le centre hospitalier capable de réaliser une telle intervention. Par exemple, dans le Nord-Pas-De-Calais, seul le CHU de Lille est en capacité de réaliser l'intervention de thrombectomie mécanique.

Une telle prise en charge doit être possible 24 heures sur 24 et 7 jours sur 7.

Elle nécessite donc une équipe dédiée avec du personnel spécialisé ; ce personnel est donc le plus souvent sous astreinte, ou de garde, et n'est pas forcément sur site.

Le Demandeur soumet ici que deux tiers de ces interventions sont pratiquées en période de garde ou d'astreinte.

Aujourd'hui, il n'existe pas de solution de synchronisation pour la prise en charge de ces patients victimes d'AVC.

Dès l'alerte AVC, le SAMU dépêche un véhicule de secours pour récupérer le patient.

Ce transfert pré-hospitalier est réalisé vers un établissement de santé disposant d'une unité neurovasculaire (UNV) ou vers un centre de télémédecine.

Par téléphone, le SAMU prévient les professionnels de santé de l'établissement de santé qu'un patient va arriver. Toutefois, les professionnels de santé ne savent pas quand et à quel moment ils doivent se mobiliser, ainsi que les autres ressources humaines et techniques de l'établissement de santé, pour être synchronisés par rapport au patient et éviter toute perte de temps.

Le Demandeur soumet par contre ici qu'une mobilisation trop prématurée de ces moyens techniques et humains dégraderait l'efficience du système de soin et la qualité de vie au travail des professionnels de santé, notamment en période de garde et d'astreinte.

À l'inverse, une mobilisation tardive de ces moyens techniques et humains entraîne des pertes de chances pour les patients.

Des examens sont ensuite réalisés sur le patient et des traitements lui sont administrés.

En France, en 2020, 7189 patients ont bénéficié d'une opération, la thrombectomie mécanique. Cette opération ne peut être réalisée que dans certains centres spécialisés, ou centre de recours, disposant d'une unité de neuroradiologie interventionnelle (NRI).

À défaut d'avoir été acheminé directement dans l'hôpital disposant d'une unité de neuroradiologie interventionnelle, le patient devra alors faire l'objet d'un transfert inter-hospitalier.

Les équipes du premier établissement de santé et de la NRI échangent alors par téléphone pour valider la prise en charge pour thrombectomie mécanique.

Lorsque cette intervention est confirmée par la NRI, l'UNV contacte le SAMU pour dépêcher un moyen de transport pour le patient. En fonction des heures de la journée, des interventions en cours et d'autres paramètres, le temps de prise en charge du patient par ce moyen de transport est variable, de sorte qu'il est impossible de déterminer à l'avance quand le patient va effectivement partir de l'UNV vers la NRI.

L'équipe de l'UNV, lorsqu'elle a le temps, prévient que le véhicule de secours part de l'UNV vers la NRI. Toutefois, cet appel est généralement oublié.

Ainsi, lors d'un transfert pré-hospitalier et d'un transfert inter-hospitalier, on peut affirmer que les professionnels de santé sont informés qu'un patient va arriver. En revanche, personne ne sait précisément à quel moment le patient va arriver.

Cette problématique est exacerbée pendant les périodes d'astreintes, lorsque les professionnels de santé qui doivent prendre en charge le patient ne sont pas dans les locaux de l'hôpital. Ils sont informés qu'un patient va arriver et doivent tout faire pour arriver en avance du patient. Pourtant ils ne savent pas quand ils doivent se préparer pour prendre en charge le patient à l'heure.

L'amélioration de la coordination des professionnels de santé, notamment dans la filière AVC, est une réelle attente.

Le Demandeur soumet qu'il n'existe pas de système permettant de synchroniser les professionnels de santé avec les patients pendant leur transfert.

De manière générale, il n'existe pas de systèmes permettant de synchroniser plusieurs personnes en temps réel pour qu'elles se rendent simultanément, ou avec un temps de décalage prédéfinis, sur le lieu d'intérêt.

Dans JP2021026649A, le moment du départ d'un deuxième véhicule est donné, et la "driving support unit" vise à ralentir le véhicule trop rapide et faire avancer plus vite le véhicule plus lent. US2019/120643A1 divulgue la synchronisation d'un véhicule ayant un malade à bord avec une ambulance. S'ils vont à l'encontre l'un de l'autre, leur point de rencontre est calculé.

### Objet de la présente invention

La présente invention vise à améliorer la situation décrite ci-dessus.

La présente invention vise notamment à remédier à au moins un des différents problèmes techniques mentionnés ci-dessus en proposant une solution pour gérer la synchronisation des arrivées d'au moins deux individus sur un même lieu d'intérêt.

L'objet de la présente invention porte, selon un premier aspect, sur un procédé de synchronisation des arrivées respectives d'au moins un premier et d'au moins un deuxième individu sur un même lieu d'intérêt à un instant t, dit instant d'arrivée, pour une intervention. Selon la présente invention, les premier et deuxième individus se situent respectivement à des premier et deuxième points de départ à un instant de départ.

Selon la présente invention, le deuxième point de départ de l'au moins un deuxième individu ne se situe pas dans une zone de proximité définie autour dudit lieu d'intérêt. On comprendra par exemple que ledit au moins un deuxième individu est chez lui, sur un autre site, ou une autre salle du même site.

Le procédé est mis en œuvre par des moyens informatiques.

Selon la présente invention, le procédé comprend les étapes suivantes :
a) une première géolocalisation dudit au moins un premier individu à l'instant de départ pour déterminer à l'aide d'une balise de géolocalisation une position géographique du premier point de départ ;
b) un premier calcul par un calculateur d'un premier temps de trajet entre le premier point de départ et le lieu d'intérêt afin d'estimer l'instant d'arrivée correspondant à l'arrivée dudit au moins un premier individu sur le lieu d'intérêt ;
c) une deuxième géolocalisation dudit au moins un deuxième individu à l'instant de départ pour déterminer une position géographique du deuxième point de départ ;
d) un deuxième calcul par ledit calculateur d'un deuxième temps de trajet entre le deuxième point de départ et le lieu d'intérêt ;
d) une détermination par ledit processeur d'un moment de départ dudit au moins un deuxième individu en fonction du deuxième temps de trajet et de l'instant d'arrivée ;
e) une génération et une émission d'un signal d'avertissement à destination d'un terminal de communication dudit au moins un deuxième individu, le signal d'avertissement contenant une information d'intervention relative à la planification de l'intervention à l'instant d'arrivée dudit au moins un premier individu sur le lieu d'intérêt et le moment de départ pour permettre audit au moins un deuxième individu de quitter le deuxième point de départ afin d'arriver de façon synchronisée sur le lieu d'intérêt avec ledit au moins un premier individu.

Grâce à la succession des étapes techniques ci-dessus, il devient possible de fournir une solution permettant la gestion des arrivées synchronisées de plusieurs individus sur un même lieu. Ceci est particulièrement intéressant dans le domaine de la santé pour optimiser la prise en charge d'un patient par un professionnel de santé dans un bloc opératoire comme c'est le cas par exemple pour les patients victime d'un AVC qui doivent subir une thrombectomie mécanique.

Avantageusement, le moment de départ est en outre déterminé en fonction d'un temps de préparation prédéterminé.

Avantageusement, le procédé selon la présente invention comporte :
- une interrogation via un serveur central d'un agenda électronique pour identifier au moins une disponibilité du lieu d'intérêt ;
- une planification par un processeur d'une intervention sur le lieu d'intérêt en déterminant, parmi ladite au moins une disponibilité du lieu d'intérêt, la disponibilité du lieu d'intérêt la plus proche de l'instant d'arrivée estimée.

Avantageusement, l'étape de planification comprend une réservation dans l'agenda électronique d'une intervention selon la disponibilité du lieu d'intérêt la plus proche de l'instant d'arrivée estimée.

Avantageusement, le procédé selon la présente invention comporte, préalablement aux première et/ou deuxième étapes de calcul, une interrogation d'un serveur de trafic déporté pour récupérer des données de trafic entre le lieu d'intérêt et respectivement le premier et/ou le deuxième points de départ.

Avantageusement, les première et/ou deuxième étapes de calcul prennent en considération lesdites données de trafic.

Avantageusement, les première et/ou deuxième étapes de calcul mettent en œuvre un apprentissage du type Machine Learning pour calculer respectivement les premier et/ou deuxième temps de trajet.

Avantageusement, le procédé selon la présente invention comprend un suivi de la position dudit au moins un premier individu sur le premier trajet entre le premier point de départ et le lieu d'intérêt pour ré-évaluer en continu ou de façon périodique l'arrivée dudit au moins premier individu sur le lieu d'intérêt.

Selon un deuxième aspect, l'objet de la présente invention concerne un programme d'ordinateur qui comporte des instructions adaptées pour l'exécution des étapes du procédé selon le premier aspect de l'invention, ceci notamment lorsque le programme d'ordinateur est exécuté par au moins un processeur.

Un tel programme d'ordinateur peut utiliser n'importe quel langage de programmation, et être sous la forme d'un code source, d'un code objet, ou d'un code intermédiaire entre un code source et un code objet, tel que dans une forme partiellement compilée, ou dans n'importe quelle autre forme souhaitable.

Selon un troisième aspect, l'objet de la présente invention concerne un support d'enregistrement lisible par un ordinateur sur lequel est enregistré un programme d'ordinateur comprenant des instructions pour l'exécution des étapes du procédé selon le premier aspect de l'invention.

D'une part, le support d'enregistrement peut être n'importe quel entité ou dispositif capable de stocker le programme. Par exemple, le support peut comporter un moyen de stockage, tel qu'une mémoire ROM, un CD-ROM ou une mémoire ROM de type circuit microélectronique, ou encore un moyen d'enregistrement magnétique ou un disque dur.

D'autre part, ce support d'enregistrement peut également être un support transmissible tel qu'un signal électrique ou optique, un tel signal pouvant être acheminé via un câble électrique ou optique, par radio classique ou hertzienne ou par faisceau laser autodirigé ou par d'autres moyens. Le programme d'ordinateur selon l'invention peut être en particulier téléchargé sur un réseau de type Internet.

Alternativement, le support d'enregistrement peut être un circuit intégré dans lequel le programme d'ordinateur est incorporé, le circuit intégré étant adapté pour exécuter ou pour être utilisé dans l'exécution du procédé en question.

L'objet de la présente invention concerne selon un quatrième aspect un système de synchronisation des arrivées respectives d'au moins un premier et d'au moins un deuxième individu sur un même lieu d'intérêt à un instant t, dit instant d'arrivée, pour une intervention non-planifiée,
Selon la présente invention, le système comprenant des moyens configurés pour la mise en œuvre des étapes du procédé selon le premier aspect décrit ci-dessus.

Plus particulièrement, le système comprend :
a) un module de géolocalisation configuré pour déterminer une position géographique du premier point de départ dudit au moins un premier individu à l'instant de départ ;
b) un calculateur configuré pour calculer un premier temps de trajet dudit au moins un premier individu entre le premier point de départ et le lieu d'intérêt afin d'estimer l'instant d'arrivée correspondant à l'arrivée dudit au moins un premier individu sur le lieu d'intérêt ; dans lequel ledit module de géolocalisation est en outre configuré pour déterminer à l'instant de départ une position géographique du deuxième point de départ dudit au moins un deuxième individu ; et dans lequel le calculateur est en outre configuré pour calculer un deuxième temps de trajet entre le deuxième point de départ et le lieu d'intérêt.

Selon la présente invention, le système comprend en outre un processeur configuré pour :
- déterminer en fonction du deuxième temps de trajet et de l'instant d'arrivée un moment de départ dudit au moins un deuxième individu ; et
- générer et émettre un signal d'avertissement à destination d'un terminal de communication dudit au moins un deuxième individu, ledit signal d'avertissement contenant une information d'intervention relative à la planification de l'intervention à l'instant d'arrivée (t) dudit au moins un premier individu sur le lieu d'intérêt et ledit moment de départ pour permettre audit au moins un deuxième individu de quitter le deuxième point de départ afin d'arriver de façon synchronisée sur le lieu d'intérêt avec ledit au moins un premier individu.

Ainsi, par ses différentes caractéristiques techniques fonctionnelles et structurelles décrites ci-dessus, on met à disposition des professionnels de la santé un outil fiable et performant permettant de synchroniser l'arrivée du personnel médical, notamment de garde ou en astreinte, avec celle d'un patient en situation d'urgence pour une prise en charge optimale. On comprendra que la présente invention servira également à des applications pour le covoiturage ou le transport de colis en permettant la synchronisation des arrivées respectives d'au moins deux individus sur un même lieu d'intérêt.

### Brève description des figures annexées

D'autres caractéristiques et avantages de la présente invention ressortiront de la description ci-dessous, en référence aux figures annexées qui en illustrent un exemple de réalisation dépourvu de tout caractère limitatif et sur lesquelles :
[Fig.1] : la figure 1 représente de façon schématique le parcours de prise en charge d'un patient victime d'un AVC selon un exemple d'application de la présente invention ;
[Fig.2] : la figure 2 est un organigramme illustrant l'ensemble des étapes du procédé selon un exemple de réalisation de la présente invention ; et
[Fig.3] : la figure 3 représente de façon schématique un système de synchronisation selon un exemple de réalisation de la présente invention.

### Description détaillée selon un exemple de réalisation avantageux

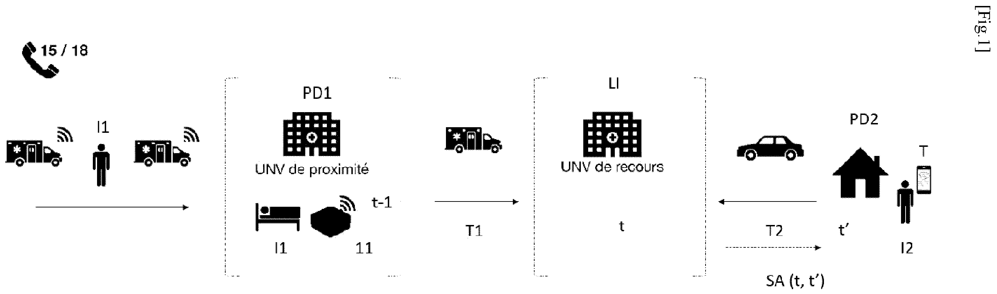

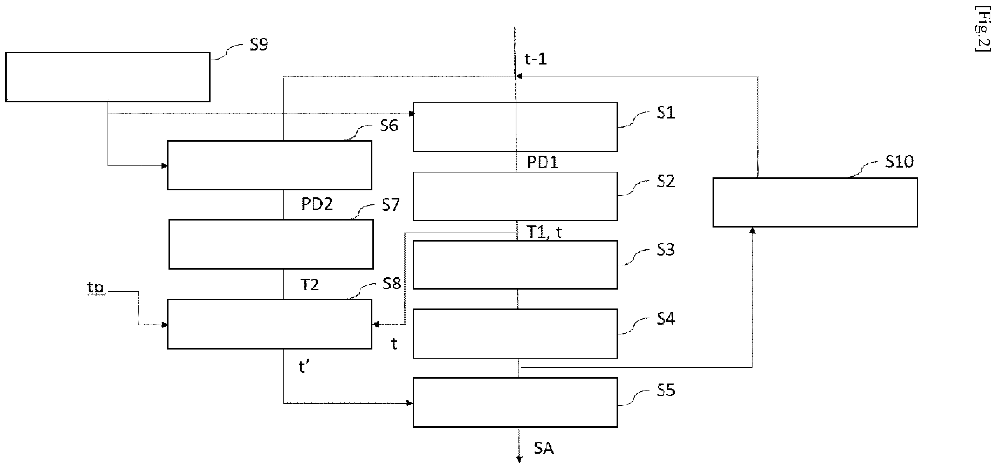

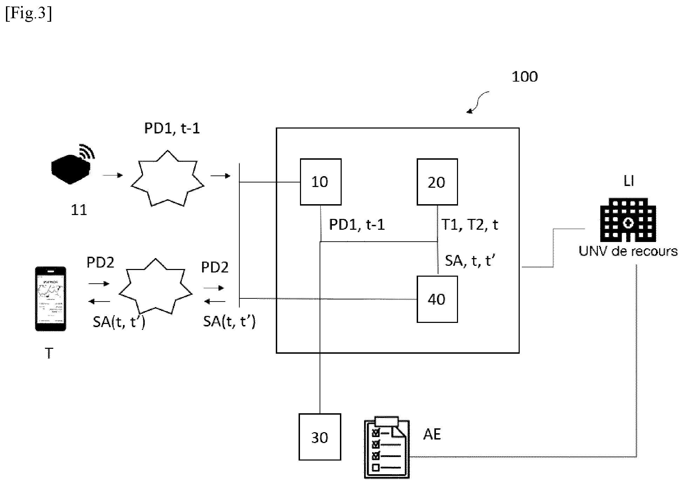

Un système de synchronisation des arrivées respectives d'un premier et d'un deuxième individus sur un même lieu d'intérêt pour une intervention ainsi que le procédé qui lui est associé vont maintenant être décrits dans ce qui va suivre en référence conjointement aux figures 1 à 3.

L'exemple décrit qui va suivre concerne la prise en charge d'un patient victime d'un AVC. Il s'agit donc ici d'une intervention d'urgence non planifiée.

On comprendra ici qu'il s'agit d'un exemple d'application parmi d'autres possibles lequel ne présente aucun caractère limitatif à la présente invention qui peut s'appliquer à d'autres domaines.

Comme expliqué en préambule, la prise en charge d'un patient victime d'un AVC est complexe et fait intervenir un grand nombre d'acteurs, ceci de l'appel au centre d'urgence (via un numéro d'urgence du type le « 15 » ou le « 18 ») jusqu'à la sortie de l'hospitalisation.

Lorsqu'il survient, l'AVC est une urgence absolue en raison de la fenêtre thérapeutique étroite (jusqu'à 6h pour une thrombectomie mécanique).

L'ensemble des professionnels de santé intervenant sur cette chaîne de soins doit donc interagir de façon coordonnée pour éviter que des retards ne s'accumulent et ne pénalisent les chances de récupération du patient.

À ce jour, il n'existe aucune solution automatisée de synchronisation.

Synchroniser sur un lieu d'intervention LI l'arrivée du praticien I2 qui va procéder à la thrombectomie avec celle du patient I1 victime de l'AVC est un des objectifs de la présente invention.

Un tel objectif est atteint par l'invention décrite ci-dessus qui prévoit un système 100 dédié pour synchroniser les arrivées respectives d'au moins deux individus I1 et I2 sur un même lieu LI.

On comprendra ici que la présente invention peut inclure plusieurs personnes comme par exemple plusieurs praticiens I2 intervenant sur un même patient.

Dans l'exemple décrit ici, le patient I1 est victime d'un AVC.

Comme illustré en figure 1, les premiers services d'urgence sont prévenus via un numéro d'urgence du type le « 15 » ou le « 18 ». Une fois arrivés sur place, les urgentistes réalisent un premier diagnostic : le patient I1 est alors orienté vers une unité neurovasculaire (UNV) via un véhicule de secours du type SMUR.

Des examens sont réalisés sur l'UNV et un premier traitement peut déjà être administré. Dans certains cas, le patient I1 peut être éligible à une thrombectomie mécanique.

Cette opération ne peut être réalisée que dans certains centres spécialisés, ou centre de recours, disposant d'une unité de neuroradiologie interventionnelle (NRI).

Cette unité NRI est par la suite référencée LI. C'est ici le lieu d'intervention qui dispose de l'infrastructure matériel pour réaliser la thrombectomie mécanique.

Dans cet exemple, le patient I1 doit donc faire l'objet d'un transfert inter-hospitalier vers ce lieu d'intervention LI.

Il est par ailleurs souhaitable de s'assurer que le patient I1 soit pris en charge immédiatement par le praticien I2 une fois arrivé sur ce lieu d'intervention LI, ceci pour ne perdre aucune minute dans la prise en charge.

L'objectif ici est donc de synchroniser l'arrivée du patient I1 et l'arrivée du praticien I2 à l'unité de neuroradiologie interventionnelle NRI correspondant au lieu LI.

Ainsi, dans l'exemple décrit ici, une fois que le patient I1 est diagnostiqué comme victime d'un AVC et qu'il est considéré comme éligible à la thrombectomie mécanique, le personnel soignant lui place une balise de géolocalisation 11 laquelle est activée lors d'une étape d'initialisation. Une telle balise 11 peut par exemple être fixée à la cheville du patient I1 par l'infirmier(ère) urgentiste.

Une fois allumée, la balise 11 transmet régulièrement sa géolocalisation. Cette géolocalisation permet de déterminer si la balise sort d'un périmètre prédéfini de l'UNV. Ceci marque l'instant de départ t-1 du procédé et le passage de la balise en état actif.

Une fois la balise activée, il est prévu une première géolocalisation S1 du patient I1 à l'instant de départ t-1.

Cette étape S1 permet au module de géolocalisation 10 de déterminer des données de géolocalisation du type GPS fournies par la balise lesquelles correspondent à la position géographique du premier point de départ PD1 du patient, ici par exemple l'unité neurovasculaire UNV.

Dans l'exemple décrit ici, le système 100 comprend en outre un calculateur 20 qui reçoit de la balise 10 ces données de géolocalisation et calcule lors d'une étape S2 le premier temps de trajet T1 entre le premier point de départ PD1, ici l'UNV, et le lieu d'intérêt LI, ici la salle d'angiographie LI de l'UNV, afin d'estimer l'instant d'arrivée t lequel correspond à l'arrivée du patient I1 dans cette salle LI.

La détermination de cet instant d'arrivée t est appréciée.

Dans l'exemple décrit ici, le processeur 40 peut au préalable interroger lors d'une étape S9 un serveur de trafic déporté (par exemple un serveur de trafic du type Google Maps^{®} ou équivalent) pour récupérer des données de trafic entre le lieu d'intérêt et respectivement le premier point de départ PD1.

On comprend dans cet exemple que le calculateur 20 prend ici en considération lors du calcul S2 ces données de trafic récupérées.

Dans cet exemple, il est prévu de mettre en œuvre lors de ce calcul un apprentissage du type Machine Learning pour calculer le premier temps de trajet T1.

Dans cet exemple, il est également prévu de suivre lors d'une étape S10 la position du patient I1 sur le premier trajet entre le premier point de départ PD1 et le lieu d'intérêt LI pour ré-évaluer en continu ou de façon périodique l'instant d'arrivée t.

Ceci permet d'avoir une valeur fiable qui prend en considération les différents paramètres de l'arrivée du patient I1 sur le lieu LI (trafic, conditions météorologiques, etc.).

Dans l'exemple décrit ici, le système 100 comprend ensuite des moyens pour, lors d'une étape S3, interroger via un serveur central 30 d'un agenda électronique AE et identifier au moins une disponibilité du lieu d'intérêt LI.

Les disponibilités de chaque unité UNV sont en effet gérées par un agenda électronique AE qui permet de gérer le planning de l'unité UNV. Un tel agenda est administré par une personne du service concerné qui organise le planning des admissions.

Ce planning se présente par exemple sous la forme d'un tableur, chaque case correspondant à un créneau horaire avec par exemple une mention « disponible » ou « occupée ».

Cette étape S3 d'interrogation consiste dans cet exemple à faire passer un pointeur sur ce tableur pour extraire une liste de créneaux disponibles pour l'utilisation du bloc en question, une telle liste comprenant au moins une disponibilité dudit bloc LI.

Il est ensuite prévu selon la présente invention une étape S4 visant à planifier une intervention au bloc opératoire LI en déterminant, parmi les disponibilités identifiées lors de la précédente étape, la disponibilité du lieu d'intérêt la plus proche de l'instant d'arrivée estimée t.

Cette étape S4 comprend également une réservation dans l'agenda électronique AE du créneau le plus pertinent.

Une telle étape de planification S4 est mise en œuvre par un processeur 40 configuré pour planifier de façon optimisée cette planification.

Une fois l'intervention planifiée, le processeur 40 va ensuite générer et émettre à destination d'un terminal de communication T appartenant au praticien I2 un signal d'avertissement du type par exemple « push » comprenant une information d'intervention relative à la planification de l'intervention à l'instant d'arrivée t du patient I1 sur le lieu d'intérêt LI. Dans l'exemple décrit ici, il est souhaitable que le praticien I2 soit présent sur les lieux en même temps que son patient I1, ceci pour des raisons évidentes.

Un AVC peut intervenir à n'importe quel jour de la semaine à n'importe quelle heure.

Le praticien I2 est souvent sous astreinte, par exemple à domicile ou ailleurs.

Pour remédier à cette situation, le procédé comprend une deuxième étape de géolocalisation S6 laquelle vise à géolocaliser le praticien I2 à l'instant de départ t-1, une fois que la balise 11 du patient I1 est activée.

Une telle étape S6 permet alors de déterminer une position géographique du deuxième point de départ PD2, par exemple ici le domicile du praticien.

Le calculateur 20 reçoit alors ces informations et calcule lors d'une deuxième étape de calcul S7 un deuxième temps de trajet T2 entre le deuxième point de départ PD2 et le lieu d'intérêt LI.

Dans l'exemple décrit ici, cette donnée est ensuite exploité lors d'une étape S8 au cours de laquelle le processeur 40 détermine le moment de départ t' en fonction du deuxième temps de trajet T2 et de l'instant d'arrivée t pour permettre au praticien I2 de quitter le deuxième point de départ PD2 (son domicile) afin d'arriver de façon synchronisée sur le lieu d'intérêt LI avec le patient I1.

On comprendra ici que ce moment de départ t' est en outre déterminé en fonction d'un temps de préparation prédéterminé tp de l'intervention. Ce temps de préparation tp correspond par exemple à la durée nécessaire pour l'équipe médicale de s'organiser et/ou de préparer le matériel de thrombectomie et le bloc avant l'intervention en tant que telle.

Dans cet exemple, l'information d'intervention envoyée via un message du type « push » au praticien comprend ce moment de départ t'.

Le praticien I2 reçoit alors via l'application logicielle installée sur son terminal T l'information associée au top départ t' pour arriver à temps sur le lieu d'intervention LI et ainsi prendre en charge sans délai le patient I1.

Le demandeur soumet qu'une telle solution est particulièrement appréciée dans le domaine de la santé. Elle permet de résoudre de nombreux problèmes :
Le premier problème rencontré avec les solutions classiques est lié à la géolocalisation ; la géolocalisation des véhicules de secours n'est pas accessible par tous les SAMU, de sorte qu'ils ne savent pas où se trouve le patient. Classiquement, lorsqu'ils disposent de la géolocalisation des véhicules, la problématique est que les patients peuvent venir de plusieurs départements, dépendants de SAMU différents. Il existe différents systèmes utilisés par les SAMU, mais qui ne sont pas interopérables. Un SAMU ne dispose pas de la géolocalisation des véhicules des autres SAMU.

Une problématique technique est donc de savoir, avec leur accord, où se situent les professionnels de santé devant se rendre vers l'établissement de santé.

Un autre problème rencontré avec les solutions classiques est lié à la temporalité ; pour l'instant, il est difficile, voire impossible, de savoir quand le patient part vers l'établissement de santé. Le personnel soignant sait que le patient va arriver pour être pris en charge, mais ne sait pas quand. Ceci est d'autant plus problématique que le personnel soignant doit préparer le matériel d'intervention avant l'arrivée du patient.

Le problème de temporalité se retrouve également avec le temps de trajet inconnu, eu égard aux conditions de l'urgence, les éventuels bouchons ou autres imprévus sur la route ne peuvent être entièrement pris en compte.

Enfin, un autre problème rencontré avec les solutions classiques porte sur la gestion de la confidentialité ; il est en effet primordial de sécuriser les échanges d'informations entre professionnels de santé. Les professionnels ne sont pas toujours équipés de solutions respectant la réglementation (RGPD et secret professionnel notamment) et la sécurité des données.

Trop souvent, ils utilisent des solutions grand public pour partager des données confidentielles entre eux. Celles-ci ne permettent pas, notamment :
- l'authentification forte des professionnels de santé ;
- l'hébergement certifié des données de santé ;
- la non-réutilisation des données échangées à des fins commerciales.

L'ensemble des problèmes ci-dessous est résolu dans le cadre de la présente invention.

La solution apportée par la présente invention s'apparente ainsi à un véritable système automatisé couplant un dispositif de géolocalisation et un programme qui calcul l'heure de départ du professionnel de santé pour garantir l'heure d'arrivée du professionnel de santé avec un delta de temps prédéfini à l'avance sur le patient.

Cette solution peut bien évidemment gérer les arrivées synchronisées de plus de deux individus sur un même lieu, par exemple un patient et plusieurs praticiens.

On notera que cette solution technique ne vise pas uniquement le domaine de la santé. La présente invention permet également de synchroniser les arrivées respectives de plusieurs personnes, par exemple pour du covoiturage ou de la logistique.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

Il devra également être observé que les signes de références mis entre parenthèses dans les revendications qui suivent ne présentent en aucun cas un caractère limitatif ; ces signes ont pour seul but d'améliorer l'intelligibilité et la compréhension des revendications qui suivent ainsi que la portée de la protection recherchée.

## Revendications

1. Procédé de synchronisation des arrivées respectives d'au moins un premier (I1) et d'au moins un deuxième (I2) individu sur un même lieu d'intérêt (LI) à un instant d'arrivée (t) pour une intervention,
dans lequel lesdits au moins un premier (I1) et au moins un deuxième (I2) individus se situent respectivement à des premier (PD1) et deuxième (PD2) points de départ à un instant de départ (t-1),
ledit procédé mis en œuvre par des moyens informatiques comprenant les étapes suivantes :
a) une première géolocalisation (S1) dudit au moins un premier individu (I1) à l'instant de départ (t-1) pour déterminer à l'aide d'un système de géolocalisation (11) une position géographique du premier point de départ (PD1) ;
b) un premier calcul (S2) par un calculateur (20) d'un premier temps de trajet (T1) entre le premier point de départ (PD1) et le lieu d'intérêt (LI) afin d'estimer l'instant d'arrivée (t) correspondant à l'arrivée dudit au moins un premier individu (I1) sur le lieu d'intérêt (LI) ;
c) une deuxième géolocalisation (S6) dudit au moins un deuxième individu (I2) à l'instant de départ (t-1) pour déterminer une position géographique du deuxième point de départ (PD2) ;
d) un deuxième calcul (S7) par ledit calculateur (20) d'un deuxième temps de trajet (T2) entre le deuxième point de départ (PD2) et le lieu d'intérêt (LI) ;
e) une détermination (S8) par un processeur (40) d'un moment de départ (t') dudit au moins un deuxième individu (I2) en fonction du deuxième temps de trajet (T2) et de l'instant d'arrivée (t) ;
f) une génération et une émission (S5) d'un signal d'avertissement (SA) à destination d'un terminal de communication (T) dudit au moins un deuxième individu (I2), ledit signal d'avertissement (SA) contenant une information d'intervention relative à la planification de l'intervention à l'instant d'arrivée (t) dudit au moins un premier individu (I1) sur le lieu d'intérêt (LI) et ledit moment de départ (t') pour permettre audit au moins un deuxième individu (I2) de quitter le deuxième point de départ (PD2) afin d'arriver de façon synchronisée sur le lieu d'intérêt (LI) avec ledit au moins un premier individu (I1).

2. Procédé selon la revendication 1, dans lequel le moment de départ (t') est en outre déterminé lors de l'étape de détermination (S8) en fonction d'un temps de préparation prédéterminé (tp).

3. Procédé selon la revendication 1 ou 2, lequel comprend :
- une interrogation (S3) via un serveur central (30) d'un agenda électronique (AE) pour identifier au moins une disponibilité du lieu d'intérêt (LI) ;
- une planification (S4) par ledit processeur (40) d'une intervention sur le lieu d'intérêt (LI) en déterminant, parmi ladite au moins une disponibilité du lieu d'intérêt (LI), la disponibilité du lieu d'intérêt la plus proche de l'instant d'arrivée estimée (t) ;

4. Procédé selon l'une quelconque des revendications 1 à 3, lequel comporte, préalablement aux première (S2) et/ou deuxième (S7) étapes de calcul, une interrogation (S9) d'un serveur de trafic déporté pour récupérer des données de trafic entre le lieu d'intérêt et respectivement le premier (PD1) et/ou le deuxième (PD2) points de départ, et dans lequel les première (S2) et/ou deuxième (S7) étapes de calcul prennent en considération lesdites données de trafic.

5. Procédé selon l'une quelconque des revendications précédentes, dans lequel les première (S2) et/ou deuxième (S7) étapes de calcul mettent en œuvre un apprentissage du type Machine Learning pour calculer respectivement les premier (T1) et/ou deuxième (T2) temps de trajet.

6. Procédé selon l'une quelconque des revendications précédentes, dans lequel l'étape de planification (S4) comprend une réservation dans l'agenda électronique d'une intervention selon la disponibilité du lieu d'intérêt (LI) la plus proche de l'instant d'arrivée (t) estimé.

7. Procédé selon l'une quelconque des revendications précédentes, lequel comprend un suivi (S10) de la position dudit au moins un premier individu (I1) sur le premier trajet (T1) entre le premier point de départ (PD1) et le lieu d'intérêt (LI) pour ré-évaluer en continu ou de façon périodique l'arrivée dudit au moins un premier individu (I1) sur le lieu d'intérêt (LI).

8. Programme d'ordinateur comportant des instructions adaptées pour l'exécution des étapes du procédé selon l'une quelconque des revendications 1 à 7 lorsque ledit programme d'ordinateur est exécuté par au moins un processeur.

9. Support d'enregistrement lisible par un ordinateur sur lequel est enregistré un programme d'ordinateur comprenant des instructions pour l'exécution des étapes du procédé selon l'une quelconque des revendications 1 à 7.

10. Système de synchronisation (100) des arrivées respectives d'au moins un premier (I1) et d'au moins un deuxième (I2) individus sur un même lieu d'intérêt (LI) à un instant d'arrivée (t) pour une intervention,
dans lequel lesdits au moins un premier (I1) et au moins un deuxième (I2) individus se situent respectivement à des premier (PD1) et deuxième (PD2) points de départ à un instant de départ (t-1),
ledit système (100) comprenant :
a) un module de géolocalisation (10) configuré pour déterminer à l'aide d'un système de géolocalisation (11) une position géographique du premier point de départ (PD1) dudit au moins un premier individu (I1) à l'instant de départ (t-1) ;
b) un calculateur (20) configuré pour calculer un premier temps de trajet (T1) dudit au moins un premier individu (I1) entre le premier point de départ (PD1) et le lieu d'intérêt (LI) afin d'estimer l'instant d'arrivée (t) correspondant à l'arrivée dudit au moins un premier individu (I1) sur le lieu d'intérêt (LI) ;
dans lequel ledit module de géolocalisation (10) est en outre configuré pour déterminer à l'instant de départ (t-1) une position géographique du deuxième point de départ (PD2) dudit au moins un deuxième individu (I2) ; et
dans lequel le calculateur (20) est en outre configuré pour calculer un deuxième temps de trajet (T2) entre le deuxième point de départ (PD2) et le lieu d'intérêt (LI) ;
ledit système (100) comprenant en outre un processeur (40) configuré pour :
- déterminer en fonction du deuxième temps de trajet (T2) et de l'instant d'arrivée (t) un moment de départ (t') dudit au moins un deuxième individu (I2) ; et
- générer et émettre un signal d'avertissement (SA) à destination d'un terminal de communication (T) dudit deuxième individu (I2), ledit signal d'avertissement (SA) contenant une information d'intervention relative à la planification de l'intervention à l'instant d'arrivée (t) dudit au moins un premier individu (I1) sur le lieu d'intérêt (LI) et ledit moment de départ (t') pour permettre audit au moins un deuxième individu (I2) de quitter le deuxième point de départ (PD2) afin d'arriver de façon synchronisée sur le lieu d'intérêt (LI) avec ledit au moins un premier individu (I1).

11. Système (100) selon la revendication 10 comprenant des moyens configurés pour la mise en œuvre des étapes du procédé selon l'une quelconque des revendications 2 à 7.

## Patentansprüche

1. Verfahren zum Synchronisieren der jeweiligen Ankünfte mindestens einer ersten (I1) und mindestens einer zweiten (I2) Person an einem gleichen Ort von Interesse (LI) zu einem Ankunftszeitpunkt (t) für einen Eingriff,
wobei sich die mindestens eine erste (I1) und mindestens eine zweite (I2) Person jeweils an einem ersten (PD1) und zweiten (PD2) Startpunkt zu einem Startzeitpunkt (t-1) befinden,
wobei das Verfahren von Datenverarbeitungsmitteln umgesetzt wird und die folgenden Schritte umfasst :
a) eine erste Geolokalisierung (S1) der mindestens einen ersten Person (I1) zum Startzeitpunkt (t-1), um mithilfe eines Systems zur Geolokalisierung (11) eine geografische Position des ersten Startpunkts (PD1) zu bestimmen ;
b) eine erste Berechnung (S2) einer ersten Wegzeit (T1) zwischen dem ersten Startpunkt (PD1) und dem Ort von Interesse (LI) durch einen Rechner (20), um den Ankunftszeitpunkt (t) zu schätzen, welcher der Ankunft der mindestens einen ersten Person (I1) am Ort von Interesse (LI) entspricht;
c) eine zweite Geolokalisierung (S6) der mindestens einen zweiten Person (I2) zum Startzeitpunkt (t-1), um eine geografische Position des zweiten Startpunkts (PD2) zu bestimmen;
d) eine zweite Berechnung (S7) durch den Rechner (20) einer zweiten Wegzeit (T2) zwischen dem zweiten Startpunkt (PD2) und dem Ort von Interesse (LI);
e) eine Bestimmung (S8) durch einen Prozessor (40) eines Startzeitpunkts (t') der mindestens einen zweiten Person (I2) in Abhängigkeit von der zweiten Wegzeit (T2) und dem Ankunftszeitpunkt (t);
f) ein Erzeugen und ein Senden (S5) eines Warnsignals (SA) an ein Kommunikationsendgerät (T) der mindestens einen zweiten Person (I2), wobei das Warnsignal (SA) eine Interventionsinformation bezüglich der Planung des Eingriffs zum Ankunftszeitpunkt (t) der mindestens einen ersten Person (I1) am Ort von Interesse (LI) und dem Startzeitpunkt (t') enthält, um es der mindestens einen zweiten Person (I2) zu ermöglichen, den zweiten Startpunkt (PD2) zu verlassen um synchron mit der mindestens einen ersten Person (I1) am Ort von Interesse (LI) anzukommen.

2. Verfahren nach Anspruch 1, wobei der Startzeitpunkt (t') weiter im Bestimmungsschritt (S8) in Abhängigkeit einer vorbestimmten Vorbereitungszeit (tp) bestimmt wird.

3. Verfahren nach Anspruch 1 oder 2, umfassend :
- eine Abfrage (S3) über einen zentralen Server (30) eines elektronischen Kalenders (AE), um mindestens eine Verfügbarkeit des Ortes von Interesse (LI) zu identifizieren ;
- eine Planung (S4) durch den Prozessor (40) eines Eingriffs am Ort von Interesse (LI), durch Bestimmen aus der mindestens einen Verfügbarkeit des Ortes von Interesse (LI) der Verfügbarkeit des Ortes von Interesse, die dem geschätzten Ankunftszeitpunkt (t) am nächsten liegt;

4. Verfahren nach einem der Ansprüche 1 bis 3, das vor dem ersten (S2) und/oder zweiten (S7) Berechnungsschritt eine Abfrage (S9) eines ausgelagerten Verkehrsservers zum Abrufen von Verkehrsdaten zwischen dem Ort von Interesse und dem ersten (PD1) und/oder zweiten (PD2) Startpunkt beinhaltet, und wobei der erste (S2) und/oder zweite (S7) Berechnungsschritt die Verkehrsdaten berücksichtigen.

5. Verfahren nach einem der vorstehenden Ansprüche, wobei der erste (S2) und/oder zweite (S7) Berechnungsschritt ein Lernen des Typs maschinelles Lernen umsetzen, um jeweils die erste (T1) und/oder zweite (T2) Wegzeit zu berechnen.

6. Verfahren nach einem der vorstehenden Ansprüche, wobei der Planungsschritt (S4) eine Reservierung im elektronischen Kalender eines Eingriffs gemäß der Verfügbarkeit des Ortes von Interesse (LI) umfasst, die dem geschätzten Ankunftszeitpunkt (t) am nächsten liegt.

7. Verfahren nach einem der vorstehenden Ansprüche, das eine Verfolgung (S10) der Position der mindestens einen ersten Person (I1) auf dem ersten Weg (T1) zwischen dem ersten Startpunkt (PD1) und dem Ort von Interesse (LI) umfasst, um die Ankunft der mindestens einen ersten Person (I1) am Ort von Interesse (LI) kontinuierlich oder periodisch neu zu bewerten.

8. Computerprogramm, das Anweisungen beinhaltet, die zur Ausführung der Schritte des Verfahrens nach einem der Ansprüche 1 bis 7 angepasst sind, wenn das Computerprogramm von mindestens einem Prozessor ausgeführt wird.

9. Computerlesbares Speichermedium, auf dem ein Computerprogramm gespeichert ist, das Anweisungen zur Durchführung der Schritte des Verfahrens nach einem der Ansprüche 1 bis 7 umfasst.

10. System zum Synchronisieren (100) der jeweiligen Ankünfte mindestens einer ersten (I1) und mindestens einer zweiten (I2) Person an einem gleichen Ort von Interesse (LI) zu einem Ankunftszeitpunkt (t) für einen Eingriff,
wobei sich die mindestens eine erste (I1) und mindestens eine zweite (I2) Person jeweils an einem ersten (PD1) und zweiten (PD2) Startpunkt zu einem Startzeitpunkt (t-1) befinden,
wobei das System (100) umfasst:
a) ein Geolokalisierungsmodul (10), das konfiguriert ist, um mithilfe einesGeolokalisierungssystems (11) eine geografische Position des ersten Startpunkts (PD1) der mindestens einen ersten Person (I1) zum Startzeitpunkt (t-1) zu bestimmen;
b) einen Rechner (20), der konfiguriert ist, um eine erste Wegezeit (T1) der mindestens einen ersten Person (I1) zwischen dem ersten Startpunkt (PD1) und dem Ort von Interesse (LI) zu berechnen, um den Ankunftszeitpunkt (t) zu schätzen, welcher der Ankunft der mindestens einen ersten Person (I1) am Ort von Interesse (LI) entspricht;
wobei das Geolokalisierungsmodul (10) weiter konfiguriert ist, um zum Startzeitpunkt (t-1) eine geografische Position des zweiten Startpunkts (PD2) der mindestens einen zweiten Person (I2) zu bestimmen; und
wobei der Rechner (20) weiter konfiguriert ist, um eine zweite Wegzeit (T2) zwischen dem zweiten Startpunkt (PD2) und dem Ort von Interesse (LI) zu berechnen;
wobei das System (100) weiter einen Prozessor (40) umfasst, der konfiguriert ist, um :
- in Abhängigkeit von der zweiten Wegzeit (T2) und dem Ankunftszeitpunkt (t) einen Startzeitpunkt (t') der mindestens einen zweiten Person (I2) zu bestimmen; und
- ein Warnsignal (SA) zu generieren und an ein Kommunikationsendgerät (T) der zweiten Person (I2) zu senden, wobei das Warnsignal (SA) eine Eingriffsinformation bezüglich der Planung des Eingriffs zum Ankunftszeitpunkt (t) der mindestens einen ersten Person (I1) am Ort von Interesse (LI) und dem Startzeitpunkt (t') enthält, um es der mindestens einen zweiten Person (I2) zu ermöglichen, den zweiten Startpunkt (PD2) zu verlassen um synchron mit der mindestens einen ersten Person (I1) am Ort von Interesse (LI) anzukommen.

11. System (100) nach Anspruch 10, umfassend Mittel, die zum Umsetzen der Schritte des Verfahrens nach einem der Ansprüche 2 bis 7 konfiguriert sind.

## Claims

1. Method for synchronising the respective arrivals of at least one first (I1) and at least one second (I2) individual at the same place of interest (LI) at an arrival time (t) for an intervention,
wherein said at least one first (I1) and at least one second (I2) individual are located respectively at first (PD1) and second (PD2) starting points at a starting time (t-1),
said method implemented by computer means comprising the following steps:
a) a first geolocation (S1) of said at least one first individual (I1) at the time of departure (t-1) to determine, using a geolocation system (11), a geographical position of the first starting point (PD1);
b) a first calculation (S2) by a computer (20) of a first travel time (T1) between the first starting point (PD1) and the place of interest (LI) in order to estimate the arrival time (t) corresponding to the arrival of said at least one first individual (I1) at the place of interest (LI);
c) a second geolocation (S6) of said at least one second individual (I2) at the time of departure (t-1) to determine a geographical position of the second starting point (PD2);
d) a second calculation (S7) by said computer (20) of a second travel time (T2) between the second starting point (PD2) and the place of interest (LI);
e) a determination (S8) by a processor (40) a departure time (t') of said at least one second individual (I2) as a function of the second travel time (T2) and the arrival time (t);
f) a generation and transmission (S5) a warning signal (SA) to a communication terminal (T) of said at least one second individual (I2), said warning signal (SA) containing intervention information relating to the planning of the intervention at the time of arrival (t) of said at least one first individual (I1) at the place of interest (LI) and said departure time (t') to enable said at least one second individual (I2) to leave the second departure point (PD2) in order to arrive synchronously at the place of interest (LI) with said at least one first individual (I1).

2. Method according to claim 1, wherein the starting time (t') is further determined during the determination step (S8) according to a predetermined preparation time (tp).

3. Method according to claim 1 or 2, which comprises:
- interrogating (S3) via a central server (30) an electronic calendar (AE) to identify at least one availability of the place of interest (LI);
- planning (S4) by said processor (40) an intervention at the place of interest (LI) by determining, among said at least one availability of the place of interest (LI), the availability of the place of interest closest to the estimated time of arrival (t);

4. Method according to any one of claims 1 to 3, which comprises, prior to the first (S2) and/or second (S7) calculation steps, an interrogation (S9) of a remote traffic server to retrieve traffic data between the place of interest and respectively the first (PD1) and/or second (PD2) starting points, and wherein the first (S2) and/or second (S7) calculation steps take into consideration said traffic data.

5. Method according to any one of the preceding claims, wherein the first (S2) and/or second (S7) calculation steps implement machine learning to calculate the first (T1) and/or second (T2) travel time respectively.

6. Method according to any one of the preceding claims, wherein the planning step (S4) comprises a booking in the electronic calendar of an intervention according to the availability of the nearest place of interest (LI) to the estimated time of arrival (t).

7. Method according to any one of the preceding claims, which comprises monitoring (S10) the position of said at least one first individual (I1) on the first route (T1) between the first starting point (PD1) and the place of interest (LI) to continuously or periodically re-evaluate the arrival of said at least one first individual (I1) at the place of interest (LI).

8. Computer program including instructions suitable for performing the steps of the method according to any one of claims 1 to 7 when said computer program is executed by at least one processor.

9. Computer-readable recording medium on which a computer program is recorded comprising instructions for performing the steps of the method according to any one of claims 1 to 7.

10. System (100) for synchronising the respective arrivals of at least one first (I1) and at least one second (I2) individual at the same place of interest (LI) at an arrival time (t) for an intervention,
wherein said at least one first (I1) and at least one second (I2) individual are located respectively at first (PD1) and second (PD2) starting points at a starting time (t-1), said system (100) comprising:
a) a geolocation module (10) configured to determine, using a geolocationsystem (11), a geographical position of the first starting point (PD1) of said at least one first individual (I1) at the starting time (t-1);
b) a computer (20) configured to calculate a first travel time (T1) of said at least one first individual (I1) between the first starting point (PD1) and the place of interest (LI) in order to estimate the arrival time (t) corresponding to the arrival of said at least one first individual (I1) at the place of interest (LI);
wherein said geolocation module (10) is further configured to determine at the starting time (t-1) a geographical position of the second starting point (PD2) of said at least one second individual (I2); and
wherein the calculator (20) is further configured to calculate a second travel time (T2) between the second starting point (PD2) and the place of interest (LI);
said system (100) further comprising a processor (40) configured to:
- determine according to the second travel time (T2) and the arrival time (t) a departure time (t') of said at least one second individual (I2); and
- generate and transmit a warning signal (SA) to a communication terminal (T) of said second individual (I2), said warning signal (SA) containing intervention information relating to the planning of the intervention at the time of arrival (t) of said at least one first individual (I1) at the place of interest (LI) and said departure time (t') to enable said at least one second individual (I2) to leave the second departure point (PD2) in order to arrive synchronously at the place of interest (LI) with said at least one first individual (I1).

11. System (100) according to claim 10, comprising means configured to implement the steps of the method according to any one of claims 2 to 7.
